# FASCICULE DE BREVET EUROPEEN

(11) **EP 1 297 177 B1**
(45) Date de publication et mention de la délivrance du brevet: **24.11.2004**
(21) Numéro de dépôt: 01949540.7
(22) Date de dépôt: 27.06.2001
(51) Int. Cl.: C12Q 1/04

(54) **DETECTION ET/OU DISCRIMINATION DES BACTERIES DU GENRE VIBRIO**
NACHWEIS UND/ODER UNTERSCHEIDUNG DER BAKTERIEN DER GATTUNG VIBRIO
VIBRIO BACTERIA DETECTION AND/OR DIFFERENTIATION

(30) Priorité: 27.06.2000 FR 0008237
(43) Date de publication de la demande: 02.04.2003
(73) Titulaire: Rambach, Alain, F-75006 Paris (FR)
(72) Inventeur: Rambach, Alain, F-75006 Paris (FR)
(74) Mandataire: Warcoin, Jacques
(86) Numéro de dépôt international: PCT/FR2001/002033
(87) Numéro de publication internationale: WO 2002/000922

(56) Documents cités:
- US-A- 4 308 346
- O'BRIEN M ET AL: "MODIFIED TAUROCHOLATE-TELLURITE-GELATIN AGAR FOR IMPROVED DIFFERENTIATION OF VIBRIO-SPP" JOURNAL OF CLINICAL MICROBIOLOGY, vol. 22, no. 6, 1985, pages 1011-1013, XP001003084 ISSN: 0095-1137
- CHANG TSUNG C ET AL: "Development of a latex agglutination test for the rapid identification of Vibrio parahaemolyticus." JOURNAL OF FOOD PROTECTION, vol. 57, no. 1, 1994, pages 31-36, XP001003097 ISSN: 0362-028X
- CHATTERJEE B D ET AL: "STUDIES ON THE BETA D GALACTOSIDASE ACTIVITY OF VIBRIO-PARAHAEMOLYTICUS" INDIAN JOURNAL OF MEDICAL RESEARCH, vol. 60, no. 6, 1972, pages 831-833, XP001003086 ISSN: 0019-5340
- BHATTACHARYA G ET AL: "STUDIES ON BETA GALACTOSIDASE ACTIVITY IN VIBRIO-ELTOR" CANADIAN JOURNAL OF MICROBIOLOGY, vol. 20, no. 6, 1974, pages 897-898, XP001003041 ISSN: 0008-4166
- MAUGERI T L ET AL: "Potentially pathogenic vibrios in brackish waters and mussels." JOURNAL OF APPLIED MICROBIOLOGY, vol. 89, no. 2, août 2000 (2000-08), pages 261-266, XP001003099 ISSN: 1364-5072
- KAZNOWSKI A. ACTA MICROBIOLOGICA POLONICA vol. 40, no. 1, 1991, pages 71 - 76

## Description

La présente invention concerne un milieu de culture chromogène destiné à mettre en évidence les bactéries du genre *Vibrio.*

Le genre *Vibrio* est associé au choléra (*V. cholerae*) et à plusieurs autres pathologies cliniques. En particulier, *V. parahaemolyticus* est responsable de certaines gastro-entérites aiguës, en particulier au Japon, observées après ingestion d'aliments contaminés (généralement des produits de la mer, poissons, coquillages, crustacés ou mollusques crus ou peu cuits).

La transmission du vibrion cholérique s'effectue généralement d'homme à homme (vomissements, matières fécales, sueurs), ou par l'intermédiaire d'aliments contaminés. Par ailleurs, on trouve naturellement les vibrions dans la flore microbienne des eaux côtières et estuariennes. Parmi leurs hôtes naturels, on compte les mollusques, crustacés, poissons, échinodermes, ou les planctons.

Les infections à vibrions posent un problème de santé publique, et il est donc important de disposer d'un test fiable et rapide permettant de détecter les contaminations alimentaires par ces bactéries.

Il existe aujourd'hui des milieux de culture plus ou moins sélectifs qui permettent de détecter la présence de vibrions dans des échantillons. En particulier, on utilise la gélose TCBS (Thiosulfate de sodium - Citrate de sodium - Bile de boeuf- Saccharose)(Diagnostics Pasteur, réf. 69456 ; Oxoid, réf. CM 333 ; Merck, réf. 10263). La grande majorité des entérobactéries et les entérocoques poussent peu ou pas du tout sur ce milieu par rapport aux vibrions. Un milieu de culture pour la détection de bactéries du genre Vibrio comportant un agent chromogène substrat de β-galactosidase est divulgué dans O'Brien M et al. Journal of clinical Microbiology, Vol. 22 no. 6, 1985, pages 1011-1013. Seul pour V.Cholerae l'activité de β-glucosidase est connue (Kaznowski, A et al. Acta Microbiologica Polonica, 1991, Vol. 40, no. 1, pages 71-76.

La gélose TTG (Taurocholate - Tellurite - Gélatine) est également utilisée, mais est relativement fastidieux à préparer, nécessitant des contrôles réguliers et un dosage rigoureux du tellurite de potassium.

Un des inconvénients du milieu TCBS est qu'il ne permet généralement pas de discriminer entre les différentes espèces de vibrions. Il conviendrait en effet de pouvoir identifier l'espèce de *Vibrio* présente dans l'échantillon analysé. En particulier, il serait avantageux de pouvoir identifier la présence et de discriminer *V. cholerae, V. parahaemolyticus, V. alginolyticus.* Cette dernière espèce n'est que rarement associée à des pathologies diarrhéiques, mais est probablement l'entité la plus fréquemment isolée dans les pays à climat tempéré, dans une grande diversité d'échantillons cliniques.

Afin d'améliorer l'évaluation du risque sanitaire, il convient donc de pouvoir discriminer les différents types de vibrions pouvant être présents dans des échantillons (alimentaires, fèces...), de façon plus fine que ce qui était obtenu par les milieux précédents, qui ne pouvaient que différentier les bactéries saccharose ⁺ (telles *V. alginolyticus* ou *V. cholerae*) des bactéries saccharose- (telles *V. parahaemolyticus*).

La présente invention se propose de fournir un nouveau milieu de culture, pour la détection et/ou la discrimination de bactéries du genre *Vibrio,* caractérisé en ce qu'il comporte, dans un milieu de culture de *Vibrio,* au moins un agent chromogène choisi parmi les substrats de la β-glucosidase et les substrats de la β-galactosidase.

L'agent chromogène substrat d'une des enzymes citées ci-dessus comprend un chromophore, qui est libéré par l'hydrolyse du substrat par son enzyme. Ainsi, la colonie bactérienne se colore en fonction du chromophore libéré.

De façon préférée, ledit chromophore est choisi parmi les dérivés indoxyle, halogéno-indoxyle (bromo-indoxyle, chloro-indoxyle, fluoro-indoxyle, iodo-indoxyle, dichloro-indoxyle, chloro-bromo-indoxyle, tri-chloro-indoxyle), le méthyl-indoxyle, ou hydroxy-quinoline. Des dérivés préférés sont choisis en particulier parmi les dérivés suivants : 6-chloro-indoxyle, 5-bromo-indoxyle, 3-bromo-indoxyle, 6-fluoro-indoxyle, 5-iodo-indoxyle, 4,6-dichloro-indoxyle, 6,7-dichloro-indoxyle, 5-bromo-4-chloro-indoxyle, 5-bromo-6-chloro-indoxyle, 4,6,7-trichloro-indoxyle, N-méthyl-indoxyle et 8-hydroxy-quinoline.

Ainsi, de façon préférée, le substrat de la β-glucosidase est un indoxyl-glucoside, et/ou le substrat de la β-galactosidase est un indoxyl-galactoside.

La concentration de chaque agent chromogène dans le milieu est comprise entre environ 0,02 et 0,5 g/l. Une concentration préférée est 0,1 g/l.

*V. parahaemolyticus* est décrit comme négatif pour le caractère β-glucosidase (galerie d'identification Rapid 20E de la société BioMérieux, Marcy l'Etoile, France), et comme positif pour le caractère β-galactosidase (galerie d'identification API 20 NE de la société BioMérieux, op. cit.).

De façon surprenante, l'invention montre que *V. parahaemolyticus* peut présenter un phénotype inverse pour ces deux caractères sur milieu solide. Ainsi, l'utilisation d'un agent chromogène substrat pour la β-glucosidase permet de colorer les colonies sur milieu solide, alors qu'elles restent incolores lorsqu'un agent chromogène substrat pour la β-galactosidase est utilisé.

Il est toutefois difficile de distinguer et discriminer *V. parahaemolyticus* de *V. alginolyticus,* qui sont souvent trouvés dans les mêmes échantillons d'origine marine, car ces deux espèces présentent de nombreux caractères communs. Ainsi, les deux espèces sont toutes deux positives pour le caractère β-glucosidase. De façon surprenante, l'ajout de saccharose au milieu selon l'invention, en particulier à une concentration élevée, permet de faire disparaître l'activité β-glucosidase de *V*. *alginolyticus,* tout en la laissant subsister chez *V. parahaemolyticus.* Ainsi, le milieu selon l'invention permet de discriminer aisément *V. parahaemolyticus* de *V. alginolyticus,* par simple coloration ou absence de coloration des colonies présentes sur le milieu de culture.

Une concentration élevée de saccharose doit être considérée comme une concentration en saccharose supérieure à 5 g/l, de préférence, comprise entre 5 g/l et 35 g/l. Une concentration de saccharose efficace est 20 g/l.

La présente invention permet également de détecter *V. cholerae,* et de le discriminer par rapport aux deux autres espèces citées ci-dessus. En effet, *V. cholerae* présente une activité β-galactosidase, sans présenter d'activité β-glucosidase. Ainsi, l'ajout d'un agent chromogène substrat pour la β-galactosidase dans un milieu de culture des *Vibrio* permet de distinguer *V. cholerae* des autres vibrions, par coloration des colonies formées.

Afin de pouvoir discriminer les différentes bactéries en une étape, il est donc intéressant d'utiliser un milieu de culture qui comporte à la fois un agent chromogène substrat de la β-galactosidase et un agent chromogène substrat de la β-glucosidase.

En effet, l'ajout de saccharose dans le milieu de culture ne fait pas disparaître le caractère β-galactosidase de *V. cholerae.* Ce résultat est d'autant plus surprenant qu'une inhibition est observée pour *V. alginolyticus,* qui présente le même caractère saccharose⁺ que *V. cholerae.*

Ainsi, l'utilisation d'un milieu de culture comportant à la fois un agent chromogène substrat de la β-galactosidase et un agent chromogène substrat de la β-glucosidase permet de différentier *V. parahaemolyticus* (mauve lorsque le 5-bromo-6-chloro-3-indoxyl-β-glucoside est utilisé), *V. cholerae* (bleu lorsque le 5-bromo-4-chloro-3-indoxyl-β-galactoside est utilisé), et *V. alginolyticus* (incolore). Un tel milieu selon l'invention permet donc la détection des deux premières souches mentionnées ci-dessus et la discrimination de ces trois souches différentes de vibrions mentionnées ci-dessus, alors que les milieux de l'art antérieur ne permettent d'obtenir qu'une différentiation en deux catégories (saccharose⁺ et saccharose⁻).

La présente invention est également dirigée vers un procédé de détection et/ou de discrimination des bactéries du genre *Vibrio* dans un échantillon, caractérisé en ce qu'il comprend les étapes suivantes :
a. inoculer un milieu de culture selon l'invention avec ledit échantillon ou un inoculum dérivé de l'échantillon,
b. détecter la présence de bactéries du genre *Vibrio* sur ledit milieu de culture,
c. de façon optionnelle, différentier *V. cholerae, V. parahaemolyticus,* et *V. alginolyticus* présents sur ledit milieu de culture.

L'utilisation de ces milieux est généralement précédée d'une étape d'enrichissement, par des méthodes connues de l'homme du métier. On peut utiliser en particulier de l'eau peptonée alcaline à 1 g % de NaCl (pH 8,6), ou de l'eau peptonée alcaline à 3 g % de NaCl.

Divers facteurs sélectifs pour *Vibrio* bien connus de l'homme du métier peuvent être ajoutés dans le milieu de la présente invention. Ainsi, la présence d'un pH alcalin, de thiosulfate, de citrate, de bile, de sel biliaire, de magnésium, de calcium, de teepol, de SDS, de Polyximine, de colistine, de tellurite... permettent d'améliorer les propriétés du milieu selon l'invention, et d'isoler les *Vibrio* de manière satisfaisante.

### EXEMPLES

### Exemple 1

Un milieu préféré pour la mise en oeuvre de l'invention comprend (pour un litre) :
- Agar 15 g
- Peptone 15 g
- Extrait de levure 3 g
- NaCl 10 g
- 5-bromo-6-chloro-3-indoxyl-β-glucoside 0,1 g
- 5-bromo-4-chloro-3-indoxyl-β-galactoside 0,1 g
- Saccharose 20 g
- Glucose 1 g
- Lactose 0,1 g
- IPTG 0,04 g
- Trizma Base 5 g
- Citrate de sodium 10 g
- Thiosulfate de sodium 10 g
- Cholate de sodium 3 g

### Exemple 2

L'étalement de bactéries sur différents milieux a donné les résultats suivants.

| | TCBS | BTB-Teepol | Milieu de l'invention (Exemple 1) |
|---|---|---|---|
| *V. cholerae* | Jaune | Jaune | Bleu |
| *V. alginolyticus* | Jaune | Jaune | Incolore |
| *V. parahaemolyticus* | Vert | Bleu-vert | Mauve |

L'utilisation du milieu selon l'invention permet donc de discriminer les trois espèces de vibrions, par rapport aux milieux de l'art antérieur.

## Revendications

1. Milieu de culture pour la détection de bactéries du genre *Vibrio* choisies dans le groupe comprenant : *V. cholerae* et *V. parahaemolyticus* et/ou la discrimination entre au moins deux des bactéries du genre Vibrio choisies dans le groupe comprenant : *V. cholerae, V. parahaemolyticus et V. alginolyticus,* **caractérisé en ce qu'**il comporte, dans un milieu de culture de *Vibrio,* au moins un agent chromogène substrat de la β-glucosidase et au moins un agent chromogène substrat de la β-galactosidase.

2. Milieu de culture selon la revendication 1, **caractérisé en ce qu'**il contient en outre une concentration de saccharose supérieure à 5 g/l de préférence comprise entre 5 g/l et 35 g/l.

3. Milieu de culture selon l'une des revendications 1 et 2, **caractérisé en ce que** ledit agent chromogène libère, par hydrolyse, un chromophore choisi parmi les dérivés indoxyle, halogéno-indoxyle (bromo-indoxyle, chloro-indoxyle, fluoro-indoxyle, iodo-indoxyle, dichloro-indoxyle, chloro-bromo- indoxyle, tri-chloro-indoxyle), méthyl-indoxyle, ou hydroxy-quinoline, en particulier les dérivés suivants : 6-chloro-indoxyle, 5-bromo-indoxyle, 3-bromo-indoxyle, 6- fluoro-indoxyle, 5-iodo-indoxyle, 4,6-dichloro-indoxyle, 6,7-dichloro-indoxyle, 5-bromo-4-chloro-indoxyle, 5-bromo-6-chloro-indoxyle, 4,6,7-trichloro-indoxyle, N-méthyl-indoxyle ou 8-hydoxy-quinoline.

4. Milieu de culture selon l'une des revendications 1 à 3, **caractérisé en ce que** ledit substrat de la β-glucosidase est un indoxyl-glucoside.

5. Milieu de culture selon la revendication 4, **caractérisé en ce que** le substrat de la β-glucosidase est le 5-bromo-6-chloro-3-indoxyl-β-glucoside.

6. Milieu de culture selon l'une des revendications 1 à 3, **caractérisé en ce que** ledit substrat de la β-galactosidase est un indoxyl-galactoside.

7. Milieu de culture selon la revendication 6, **caractérisé en ce que** le substrat de la β-galactosidase est le 5-bromo-4-chloro-3-indoxyl-β-galactoside.

8. Milieu de culture selon l'une des revendications 1 à 7, **caractérisé en ce qu'**il comprend (pour un litre) :
- Agar 15 g
- Peptone 15 g
- Extrait de levure 3 g
- NaCl 10 g
- 5-bromo-6-chloro-3-indoxyl-β-glucoside 0,1 g
- 5-bromo-4-chloro-3-indoxyl-β-galactoside 0;1 g
- Saccharose 20 g
- Glucose 1 g
- Lactose 0,1 g
- IPTG 0,04 g
- Trizma Base 5 g
- Citrate de sodium 10 g
- Thiosulfate de sodium 10 g
- Cholate de sodium 3 g

9. Utilisation d'un milieu de culture tel que défini dans l'une des revendications 1 à 8 pour la détection de bactéries du genre *Vibrio* choisies dans le groupe comprenant : *V. cholerae* et *V. parahaemolyticus* et/ou la discrimination des bactéries du genre Vibrio choisies dans le groupe comprenant: *V. cholerae, V. parahaemolyticus et V. alginolyticus.*

10. Procédé de détection de bactéries du genre *Vibrio* choisies dans le groupe comprenant : *V. cholerae* et *V. parahaemolyticus* et/ou de discrimination des bactéries du genre Vibrio choisies dans le groupe comprenant : *V. cholerae, V. parahaemolyticus et V. alginolyticus* dans un échantillon,
**caractérisé en ce qu'**il comprend les étapes suivantes :
a. inoculer un milieu de culture tel que défini dans l'une des revendications 1 à 8 avec ledit échantillon ou un inoculum dérivé de l'échantillon,
b. détecter la présence desdites bactéries sur ledit milieu de culture.

## Patentansprüche

1. Kulturmedium für den Nachweis von Bakterien der Gattung *Vibrio,* welche ausgewählt werden in der Gruppe, umfassend: *V*. *cholerae* und *V. parahaemolyticus,* und/oder für die Unterscheidung zwischen wenigstens zwei der Bakterien der Gattung Vibrio, welche ausgewählt werden in der Gruppe, umfassend:.*V*. *cholerae, V. parahaemolyticus und V. alginolyticus,* **dadurch gekennzeichnet, dass** es in einem Kulturmedium für *Vibrio* wenigstens ein chromogenes Substrat-Mittel der β-Glucosidase und wenigstens ein chromogenes Substrat-Mittel der β-Galactosidase umfasst.

2. Kulturmedium nach Anspruch 1, **dadurch gekennzeichnet, dass** es außerdem eine Saccharosekonzentration über 5 g/l, vorzugsweise zwischen 5 g/l und 35 g/l umfasst.

3. Kulturmedium nach einem der Ansprüche 1 und 2, **dadurch gekennzeichnet, dass** das chromogene Mittel durch Hydrolyse ein Chromophor freisetzt, welches ausgewählt wird unter den Indoxyl-, Halogenindoxyl- (Bromindoxyl-, Chlorindoxyl-, Fluorindoxyl-, Iodindoxyl-, Dichlorindoxyl-, Chlorbromindoxyl-, Trichlorindoxyl-), Methylindoxyl- oder Hydroxychinolinderivaten, insbesondere den folgenden Derivaten: 6-Chlorindoxyl, 5-Bromindoxyl, 3-Bromindoxyl, 6-Fluorindoxyl, 5-Iodindoxyl, 4,6-Dichlorindoxyl, 6,7-Dichlorindoxyl, 5-Brom-4-chlorindoxyl, 5-Brom-6-chlorindoxyl, 4,6,7-Trichlorindoxyl, N-Methylindoxyl oder 8-Hydroxychinolin.

4. Kulturmedium nach einem der Ansprüche 1 bis 3, **dadurch gekennzeichnet, dass** das Substrat der β-Glucosidase ein Indoxylglucosid ist.

5. Kulturmedium nach Anspruch 4, **dadurch gekennzeichnet, dass** das Substrat der β- Glucosidase 5-Brom-6-chlor-3-indoxyl-β-glucosid ist.

6. Kulturmedium nach einem der Ansprüche 1 bis 3, **dadurch gekennzeichnet, dass** das Substrat der β-Galactosidase ein Indoxylgalactosid ist.

7. Kulturmedium nach Anspruch 6, **dadurch gekennzeichnet, dass** das Substrat der β-Galactosidase 5-Brom-4-chlor-3-indoxyl-β-galactosid ist.

8. Kulturmedium nach einem der Ansprüche 1 bis 7, **dadurch gekennzeichnet, dass** es umfasst (für einen Liter):
- Agar 15 g
- Pepton 15 g
- Hefeextrakt 3 g
- NaCl 10 g
- 5-Brom-6-chlor-3-indoxyl-β-glucosid 0,1 g
- 5-Brom-4-chlor-3-indoxyl-β-galactosid 0,1 g
- Saccharose 20 g
- Glucose 1 g
- Lactose 0,1 g
- IPTG 0,04 g
- Trizma-Base 5 g
- Natriumcitrat 10 g
- Natriumthiosulfat 10 g
- Natriumcholat 3 g.

9. Verwendung eines Kulturmediums, wie in einem der Ansprüche 1 bis 8 definiert, für den Nachweis von Bakterien der Gattung *Vibrio,* welche ausgewählt werden in der Gruppe, umfassend: *V. cholerae* und *V. parahaemolyticus,* und/oder für die Unterscheidung der Bakterien der Gattung Vibrio, welche ausgewählt werden in der Gruppe, umfassend: *V. cholerae, V. parahaemolyticus und V. alginolyticus.*

10. Verfahren zum Nachweis von Bakterien der Gattung *Vibrio*, welche ausgewählt werden in der Gruppe, umfassend: *V. cholerae* und *V. parahaemolyticus*, und/oder zur Unterscheidung der Bakterien der Gattung Vibrio, welche ausgewählt werden in der Gruppe, umfassend: *V. cholerae*, *V. parahaemolyticus und V. alginolyticus,* in einer Probe,
**dadurch gekennzeichnet, dass** es die folgenden Schritte umfasst:
a. ein Kulturmedium, wie in einem der Ansprüche 1 bis 8 definiert, mit der Probe oder einem von der Probe abgeleiteten Inokulum anzuimpfen,
b. die Anwesenheit der Bakterien auf dem Kulturmedium nachzuweisen.

## Claims

1. Culture medium for detecting bacteria of the Vibrio genus chosen from the group comprising: *V. cholerae* and *V*. *parahaemolyticus* and/or for differentiating between at least two of the bacteria of the *Vibrio* genus chosen from the group comprising: *V. cholerae, V. parahaemolyticus* and *V. alginolyticus,* **characterized in that** it comprises, in a Vibrio culture medium, at least one chromogenic agent which is the substrate for β-glucosidase and at least one chromogenic agent which is the substrate for β-galactosidase.

2. Culture medium according to Claim 1, **characterized in that** it also contains a concentration of sucrose of greater than 5 g/l, preferably between 5 g/l and 35 g/l.

3. Culture medium according to either of Claims 1 and 2, **characterized in that** said chromogenic agent releases, by hydrolysis, a chromophore chosen from indoxyl, haloindoxyl (bromoindoxyl, chloroindoxyl, fluoroindoxyl, iodoindoxyl, dichloroindoxyl, chlorobromoindoxyl, trichloroindoxyl), methylindoxyl or hydroxyquinoline derivatives, in particular the following derivatives: 6-chloroindoxyl, 5-bromoindoxyl, 3-bromoindoxyl, 6-fluoroindoxyl, 5-iodoindoxyl, 4, 6-dichloroindoxyl, 6,7-dichloroindoxyl, 5-bromo-4-chloroindoxyl, 5-bromo-6-chloroindoxyl, 4,6,7-trichloroindoxyl, N-methylindoxyl or 8-hydroxyquinoline.

4. Culture medium according to one of Claims 1 to 3, **characterized in that** said β-glucosidase substrate is an indoxylglucoside.

5. Culture medium according to Claim 4, **characterized in that** the β-glucosidase substrate is 5-bromo-6-chloro-3-indoxyl-β-glucoside.

6. Culture medium according to one of Claims 1 to 3, **characterized in that** said β-galactosidase substrate is an indaxylgalactoside.

7. Culture medium according to Claim 6, **characterized in that** the β-galactosidase substrate is 5-bromo-4-chloro-3-indoxyl-β-galactoside.

8. Culture medium according to one of Claims 1 to 7, **characterized in that** it comprises (per litre):
- Agar 15 g
- Peptone 15 g
- Yeast extract 3 g
- NaCl 10 g
- 5-Bromo-6-chloro-3-indoxyl-β-glucoside 0.1 g
- 5-Bromo-4-chloro-3-indoxyl-β-galactoside 0.1 g
- Sucrose 20 g
- Glucose 1 g
- Lactose 0.1 g
- IPTG 0.04 g
- Trizma base 5 g
- Sodium citrate 10 g
- Sodium thiosulphate 10 g
- Sodium cholate 3 g

9. Use of a culture medium as defined in one of Claims 1 to 8, for detecting bacteria of the *Vibrio* genus chosen from the group comprising: *V. cholerae* and *V*. *parahaemolyticus* and/or for differentiating bacteria of the *Vibrio* genus chosen from the group comprising: *V. cholerae, V. parahaemolyticus* and *V*. *alginolyticus.*

10. Method for detecting bacteria of the *Vibrio* genus chosen from the group comprising: *V. cholerae* and *V. parahaemolyticus* and/or for differentiating bacteria of the *Vibrio* genus chosen from the group comprising: *V. cholerae, V. parahaemolyticus* and *V. alginolyticus* in a sample, **characterized in that** it comprises the following steps:
a. inoculating a culture medium as defined in one of Claims 1 to 8 with said sample or an inoculum derived from the sample,
b. detecting the presence of said bacteria on said culture medium.
